# EUROPEAN PATENT APPLICATION

(11) **EP 2 123 281 A1**
(43) Date of publication of application: **25.11.2009**
(21) Application number: 08710632.4
(22) Date of filing: 30.01.2008
(51) Int. Cl.: A61K 31/661, A61P 25/28

(54) **COMPOSITION FOR PREVENTION OR TREATMENT OF DISEASE ASSOCIATED WITH AMYLOIDOSIS THROUGH INHIBITION OF AGGREGATION AMYLOID PROTEIN AND THROUGH PROMOTION OF DEGRADATION OF AMYLOID PROTEIN**

(30) Priority: 30.01.2007 JP 2007018988
(71) Applicant: TOHOKU UNIVERSITY, Sendai-shi Miyagi 980-8577 (JP); PROJECT M CO., LTD., Sendai-shi, Miyagi 980-8579 (JP)
(72) Inventor: MIYAZAWA, Teruo, Sendai-shi Miyagi 980-8577 (JP); NAKAGAWA, Kiyotaka, Sendai-shi Miyagi 980-8577 (JP); KARIYA, Jun, Sendai-shi Miyagi 9808579 (JP)
(74) Representative: Schwahn, Hartmut
(86) International application number: PCT/JP2008/051373
(87) International publication number: WO 2008/093709

(57) **Abstract**

It is an object of the present invention to provide a means for promoting amyloid protein degradation and a means for inhibiting amyloid protein aggregation.

The present invention relates to a composition for promoting amyloid protein degradation, a composition for inhibiting amyloid protein aggregation, and a composition for preventing or treating diseases associated with amyloidosis, each of which comprises a plasmalogen as an active ingredient.

## Description

### Technical Field

The present invention relates to a composition for promoting amyloid protein degradation, a composition for inhibiting amyloid protein aggregation, and a composition for preventing or treating diseases associated with amyloidosis.

### Background Art

Amyloidosis is a disease in which abnormal proteins called amyloid proteins accumulate in various tissues and organs and inhibit the functions of normal tissues and organs. In recent years, "anti-amyloid therapies" for controlling amyloid protein deposition have been expected to be used as essential therapies for forms of amyloidosis, including Alzheimer's disease. Anti-amyloid therapies can be classified into three groups based on the following purposes for target disease stages: inhibition of amyloid protein generation; inhibition of amyloid protein deposition; and removal of amyloid proteins.

Plasmalogens are phospholipids that are specifically found to be abundantly contained in brain neurons. Plasmalogens have been predicted to be involved in brain signal transmission and to function as antioxidants in brains. Recently, a finding on antioxidant effects of plasmalogens has been reported. Effects of plasmalogens on diseases associated with aging- and oxidation-related disorders have been reported. Also, it has been reported that plasmalogen amounts and membrane critical temperatures in Alzheimer's disease patients are lower than those in healthy individuals (Non-Patent Document 1). Further, it has been reported that plasmalogens have effects of preventing neuron death in culture cell systems (Patent Document 1).
Patent Document 1: JP Patent Publication (Kokai) No. 2004-026803 A
Non-Patent Document 1: J. Neurochem. Res., 70, 2533-2538

### Disclosure of the Invention

It is an object of the present invention to provide a means for promoting amyloid protein degradation and a means for inhibiting amyloid protein aggregation.

As a result of intensive studies in order to achieve the above object, the present inventors have found that plasmalogens have effects of inhibiting amyloid protein aggregation and promoting amyloid protein degradation. This has led to the completion of the present invention.

Specifically, the present invention encompasses the following inventions.
(1) A composition for promoting amyloid protein degradation, comprising a plasmalogen as an active ingredient.
(2) A composition for inhibiting amyloid protein aggregation, comprising a plasmalogen as an active ingredient.
(3) The composition according to (1) or (2), comprising a plasmalogen having a glycerol skeleton comprising an ester-linked docosahexaenoic acid at the sn-2 position.
(4) A composition for preventing or treating diseases associated with amyloidosis, comprising a plasmalogen as an active ingredient.

The present invention provides a composition useful for inhibiting amyloid protein aggregation and promoting amyloid protein degradation.

This description includes part or all of the contents as disclosed in the description and/or drawings of Japanese Patent Application No. 2007-018988, which is a priority document of the present application.

### Best Mode for Carrying Out the Invention

Amyloidosis is characterized by accumulation of a variety of insoluble fibrous proteins in tissues. Fibrous proteins that constitute accumulated substances or deposits are referred to as "amyloid proteins." There are a variety of specific proteins or peptides present in deposits. Various types of amyloid proteins have common features in that they are formed into fibers and contain many β sheets as a secondary structure. Therefore, in the present invention, the term "amyloid protein" especially refers to amyloid β protein.

Amyloidosis is roughly classified into the following four types: (1) primary amyloidosis that is observed with amyloid protein accumulation, which results in abnormalities in plasmacytes, and is sometimes associated with multiple myeloma; (2) secondary amyloidosis that is induced by a disease such as tuberculosis or rheumatoid arthritis involving persistent inflammation and is observed with amyloid protein accumulation in the spleen, the kidneys, the lymph nodes, and the like; (3) hereditary amyloidosis that is genetic amyloidosis observed with amyloid protein accumulation in the nervous system, the heart, the kidneys, and the like; and (4) aging-induced amyloidosis that is observed with amyloid protein accumulation in the heart and the brain due to aging.

The present inventors have found that plasmalogens have activity of promoting amyloid protein degradation and activity of inhibiting amyloid protein aggregation. Therefore, in one embodiment, the present invention relates to a composition for promoting amyloid protein degradation and a composition for inhibiting amyloid protein aggregation, each of which comprises a plasmalogen as an active ingredient.

A plasmalogen is an ether phospholipid that is referred to as 1-alkenyl-2-acyl-sn-glycero-3-phosphoric acid wherein the sn-1 position of glycerol binds a vinyl ether linkage and the sn-3 position of glycerol binds a phosphoric acid to which choline or ethanolamine is linked.

In the present invention, a plasmalogen is preferably a glycerophospholipid having a glycerol skeleton comprising an ester-linked long-chain fatty acid at the sn-2 position and a vinyl-ether-linked alkyl group at the sn-1 position. Preferably, an ester-linked long-chain fatty acid at the sn-2 position is a long-chain unsaturated fatty acid. Herein, a long-chain unsaturated fatty acid refers to a fatty acid having a carbon number of 16 or more, preferably 18 to 26, and more preferably 20 to 24 and having an unsaturated linkage number of 1 or more, preferably 2 or more, and more preferably 2 to 6. Preferably, such a fatty acid is a straight chain fatty acid. Herein, specific examples of an ester-linked long-chain unsaturated fatty acid at the sn-2 position include oleic acid (18:1), linoleic acid (18:2), linolenic acid (18:3), icosenoic acid (20:1), icosadienoic acid (20:2), icosatrienoic acid (20:3), arachidonic acid (20:4), icosapentaenoic acid (20:5), docosadienoic acid (22:2), docosatrienoic acid (22:3), docosatetraenoic acid (22:4), docosapentaenoic acid (22:5), and docosahexaenoic acid (22:6). Preferably, the ester-linked long-chain unsaturated fatty acid is arachidonic acid or docosahexaenoic acid. A vinyl-ether-linked alkyl group at the sn-1 position is an alkyl group having a carbon number of 8 or more, preferably 10 to 26, and more preferably 12 to 24. Specific examples thereof include a stearyl group and a hexadecanoyl group. In addition, plasmalogens include ethanolamine plasmalogen, choline plasmalogen, serine plasmalogen, and inositol plasmalogen that have, as base portions, esters with ethanolamine, choline, serine, and inositol, respectively. Preferably, the vinyl-ether-linked alkyl group is ethanolamine plasmalogen. A combination of different types of plasmalogens may be used.

In the present invention, it is preferable to use, as a plasmalogen, a compound represented by the following formula (1): 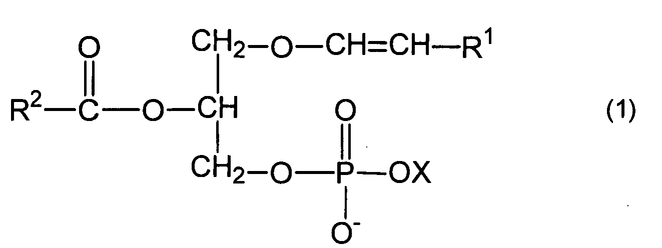 (where
R¹ represents an alkyl group having a carbon number of 8 or more, preferably 10 to 26, and more preferably 12 to 24;
R² represents an aliphatic group and preferably a straight chain aliphatic group having a carbon number of 16 or more, preferably 18 to 26, and more preferably 20 to 24, and having 1 or more, preferably 2 or more, and more preferably 2 to 6 of unsaturated linkage(s); and
X represents -CH₂CH₂N⁺H₃. -CH₂CH₂N⁺ (CH₃)₃, -CH₂CH₂N⁺H₃COO⁻ or -C₆H₁₁O₅ and preferably CH₂CH₂N⁺H₃).

It is particularly preferable to use, as a plasmalogen, a compound comprising an ester-linked docosahexaenoic acid at the sn-2 position, which is represented by the following formula (2): 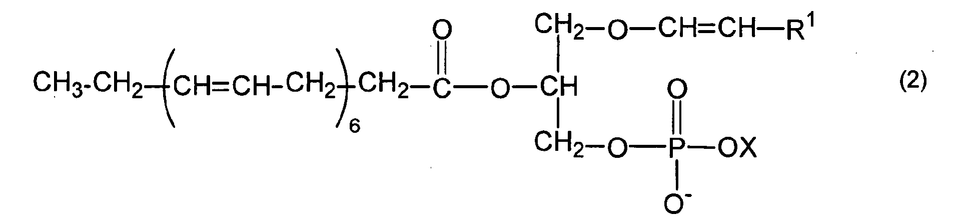 (where R¹ and X are defined as in formula (1)).

Both natural and synthetic plasmalogens may be used. Large amounts of plasmalogens are contained, in particular, in animal organs (particularly in brains, nerve tissue, cardiac muscle, skeletal muscle, and red blood cells), anaerobic bacteria, and germinating peas and soybeans. Plasmogens can be extracted from the above raw materials. Extraction solvents are not particularly limited. Extraction solvents selected from the following examples can be used: hexane, heptane, benzene, dichloromethane, chloroform, acetone, acetonitrile, cyclohexane, toluene, esters such as ethyl acetate, ethers such as 1,4-dioxane and tetrahydrofuran, lower alcohols having carbon numbers of 1 to 6 such as methanol, ethanol, and isopropanol, mixtures thereof, and mixtures comprising any of the above examples and water. An obtained plasmalogen-containing extract can be purified according to need by column chromatography using a filler for a normal-phase, reverse-phase, or ion-exchange column, by high-performance liquid chromatography, by enzyme treatment, or in a like manner.

There is a concern that docosahexaenoic acid, which has been reported to be effective against dementia, might cause *in vivo* peroxidation if it is administered. Plasmalogens, on the other hand, do not cause an *in vivo* peroxidation reaction, even in a case in which a compound comprising ester-linked docosahexaenoic acid that is represented by formula (2) is used as a plasmalogen. This is probably because a plasmalogen represented by formula (2) is less likely to be oxidized than docosahexaenoic acid itself and thus it is unlikely to serve as an initiator for an *in vivo* peroxidation reaction. For prevention and treatment of dementia such as Alzheimer's disease, it is essential to use an agent comprising an active ingredient that is appropriate for long-term administration. Plasmalogens are very advantageous in that they are unlikely to be oxidized and are appropriate for long-term administration.

The present inventors have found that plasmalogens have activity of promoting amyloid protein degradation and activity of inhibiting amyloid protein aggregation. Amyloidosis can be improved by promoting amyloid protein degradation or inhibiting amyloid protein aggregation. Therefore, in one embodiment, the present invention relates to a composition comprising a plasmalogen as an active ingredient for preventing or treating diseases associated with amyloidosis. In other words, the present invention relates to a composition comprising a plasmalogen as an active ingredient for preventing or treating diseases associated with amyloidosis through promotion of amyloid protein degradation and/or inhibition of amyloid protein aggregation.

Diseases associated with amyloidosis can be referred to as diseases that can be prevented or treated through amyloid protein degradation or inhibition of amyloid protein aggregation. Specific examples thereof include dementia such as Alzheimer's disease, Mediterranean fever, Muckle-Wells syndrome, idiopathic myeloma, multiple myeloma, amyloid polyneuropathy, amyloid cardiomyopathy, senile systemic amyloidsis, hereditary cerebral hemorrhage associated with amyloidosis, Down's syndrome, scrapie, Creutzfeldt-Jakob disease, isolated atrial amyloidosis, inclusion body myositis, sickle-cell anemia, Parkinson's disease, and Islets of Langerhans diabetes type II insulinoma. The composition of the present invention is appropriate for prevention and treatment of dementia and particularly Alzheimer's disease.

According to the present invention, the prevention of disease includes inhibition and slowing of disease development. Also, the treatment of disease includes cure of diseases, improvement of symptoms, and slowing of progression of symptoms.

Hereinafter, a composition for promoting amyloid protein degradation, a composition for inhibiting amyloid protein aggregation, and a composition for preventing or treating diseases associated with amyloidosis, each of which comprises a plasmalogen as an active ingredient, may each be referred to as "the composition of the present invention."

Subjects to which the composition of the present invention is administered are mammals. Herein, the term "mammals" refers to warm-blooded vertebrate animals. Examples thereof include: primates such as humans and monkeys; rodents such as mice, rats, and rabbits; pet animals such as dogs and cats; and domestic animals such as cattle, horses, and pigs. It is preferable to administer the composition of the present invention to primates and particularly humans. It is particularly preferable to administer the composition of the present invention to humans suffering from diseases associated with amyloidosis, humans diagnosed as suffering from diseases associated with amyloidosis, humans who may develop diseases associated with amyloidosis, and humans who need to be prevented from developing diseases associated with amyloidosis.

The composition of the present invention is not particularly limited. However, it can be prepared as, for example, a pharmaceutical composition or a food composition.

When the composition of the present invention is prepared as a pharmaceutical composition, it is generally prepared as a formulation comprising a plasmalogen and preferably a pharmaceutically acceptable carrier. Such a pharmaceutical composition is orally or parenterally (e.g., subcutaneously, intravenously, intra-arterially, intramuscularly, intraperitoneally, or intranasally) administered.

In general, the term "pharmaceutically acceptable carrier" refers to, for example, an inert and atoxic extender, diluent, or encapsulation material in a solid or liquid form, which does not react with an active ingredient used for the present invention. Examples thereof include solvents or dispersion media such as water, ethanol, polyol (e.g., glycerol, propylene glycol, and liquid polyethylene glycol), appropriate mixtures thereof, and plant oils.

Preferably, the above pharmaceutical composition is in a formulation form for oral administration. Examples of such formulation include tablets, granules, fine grains, powders, capsules, syrups, and liquids.

Such pharmaceutical composition may further contain an additive that is commonly used in the pharmaceutical field. Examples of such additive include excipients, binders, disintegrators, lubricants, antioxidants, colorants, and flavoring agents, which can be used according to need. In order to obtain a long-term acting sustained-release formulation, the composition of the present invention can be coated with a known retardant or the like. Examples of excipients that can be used include carboxymethylcellulose sodium, agar, light anhydrous silicic acid, gelatin, crystalline cellulose, sorbitol, talc, dextrine, starch, lactose, saccharose, glucose, mannitol, magnesium aluminometasilicate, and calcium hydrogen phosphate. Examples of binders include gum arabic, sodium alginate, ethanol, ethylcellulose, casein sodium, carboxymethylcellulose sodium, agar, purified water, gelatin, starch, traganth, lactose, hydroxycellulose, hydroxymethylcellulose, hydroxypropylcellulose, and polyvinyl pyrrolidone. Examples of disintegrators include carboxymethylcellulose, carboxymethylcellulose sodium, carboxymethylcellulose calcium, crystalline cellulose, starch, and hydroxypropyl starch. Examples of lubricants include stearic acid, calcium stearate, magnesium stearate, talc, hardened oil, sucrose fatty acid ester, and waxes. Examples of antioxidants include tocopherol, ester gallate, dibutylhydroxytoluene (BHT), butylhydroxyanisole (BHA), and ascorbic acid. Other additives and drugs such as antacids (e.g., sodium bicarbonate, magnesium carbonate, precipitated calcium carbonate, and synthetic hydrotalcite) and gastric mucosal coating agents (e.g., synthetic aluminum silicate, sucralfate, and copper chlorophylline sodium) may be added according to need.

The pharmaceutical composition may be parenterally administered in the form of a sustained-release subcutaneous implant or a target delivery system (e.g., a monoclonal antibody, a vector delivery system, an ion implantation system, a polymer matrix, a liposome, or a microsphere).

When the composition of the present invention is prepared as a food composition, the form thereof is not particularly limited. It may be in the form of a beverage, health food, or functional food. Health food and functional food can be formed into a variety of formulations. Specific examples thereof include fine grains, tablets, granules, powders, capsules, syrups, liquids, and liquid meals. A food composition in a form of formulation can be produced in a manner similar to that for a pharmaceutical formulation. For instance, it can be produced by adding an adequate excipient (e.g., starch, processed starch, lactose, glucose, or water) and using a common means. Further, such a food composition can be in the form of: a liquid food composition such as soup, juice, milk beverage, cocoa beverage, or jelly beverage; a semi-solid food composition such as pudding or yogurt; bread; sweets; noodles such as Japanese wheat noodles (*udon*); sweets such as cookies, chocolate and candy; snacks such as rice crackers; toppings for cooked rice; and spread such as butter or jam.

It is possible to add a single additive or a combination of additives to the food composition. Examples of additives include a variety of food additives such as antioxidants, flavors, a variety of esters, organic acids, organic acid salts, inorganic acids, inorganic acid salts, inorganic salts, pigments, emulsifiers, preservatives, seasonings, edulcorants, acidulants, fruit juice extracts, vegetable extracts, nectar extracts, pH adjusters, and quality stabilizers.

The administration dose of the composition of the present invention can be adequately determined depending on administration methods and patients' ages, symptoms, and the like. The daily dose per kilogram body weight is generally 0.01 to 2 mg and preferably 0.1 to 0.2 mg for oral administration.

The plasmalogen content in the composition of the present invention can be adequately determined at a level at which the above administration dose can be realized. For instance, the plasmalogen content is generally 0.35% to 70% by mass and preferably 3.5% to 7% by mass in a tablet, granule, fine grain, powder, or capsule formulation.

A functional component may be added to the composition of the present invention. Examples of functional components include vitamin C, vitamin E, vitamin B12, vitamin B2, vitamin B1, xanthophylls, carotenoids, tocotrienol, minerals (calcium, magnesium, iron, zinc, etc.), phosphatidyl choline, phosphatidyl serine, ginkgo leave extracts, chlorella, wine polyphenol, green tea catechin, theaflavin, and Perilla oil.

It is possible to promote amyloid protein degradation and/or to inhibit amyloid protein aggregation by administering the composition of the present invention in effective doses to mammals; that is to say, by administering a plasmalogen in effective doses to mammals. Accordingly, it becomes possible to prevent or treat diseases associated with amyloidosis.

Plasmalogens are safe food components that have been widely contained in meals. Therefore, plasmalogens can be advantageously used for prevention of amyloidosis and slowing of progress of symptoms of amyloidosis through nutritional guidance and the like in daily diet.

The present invention is hereafter described in greater detail with reference to the following examples, although the technical scope of the present invention is not limited thereto.

### Examples

Amyloid β1-42, which is an aggregative protein, was incubated for induction of aggregation. The degree of aggregation was determined with an assay system using a fluorescent reagent or a qualitative system using an electron microscope. Before and after the occurrence of aggregation, a plasmalogen or a phospholipid other than a plasmalogen was added to the system. Effects of a plasmalogen or a phospholipid upon amyloid protein aggregation were evaluated by experiments in Examples 1 to 6. In addition, samples and reagents used in the Examples are shown as follows: disodium hydrogen phosphate (Wako Pure Chemical Industries, Ltd.); sodium dihydrogen phosphate (Wako Pure Chemical Industries, Ltd.); amyloid β1-42 (Pepetide Institute, Inc.); thioflavine T (Sigma); plasmalogen having a glycerol skeleton comprising ester-linked arachidonic acid at the sn-2 position (Pls-AA; Avanti Polar Lipids); plasmalogen having a glycerol skeleton comprising ester-linked oleic acid at the sn-2 position (Pls-OA; Avanti Polar Lipids); plasmalogen having a glycerol skeleton comprising ester-linked docosahexaenoic acid (DHA) at the sn-2 position (Pls-DHA; Avanti Polar Lipids); phosphatidyl ethanolamine having a glycerol skeleton comprising ester-linked docosahexaenoic acid at the sn-2 position (PE-DHA; Avanti Polar Lipids); phosphatidyl choline having a glycerol skeleton comprising ester-linked docosahexaenoic acid at the sn-2 position (PC-DHA, Avanti Polar Lipids); and a fluorescent plate reader (Molecular Devices).

In addition, the plasmalogens used in the Examples are represented by the following formulae.

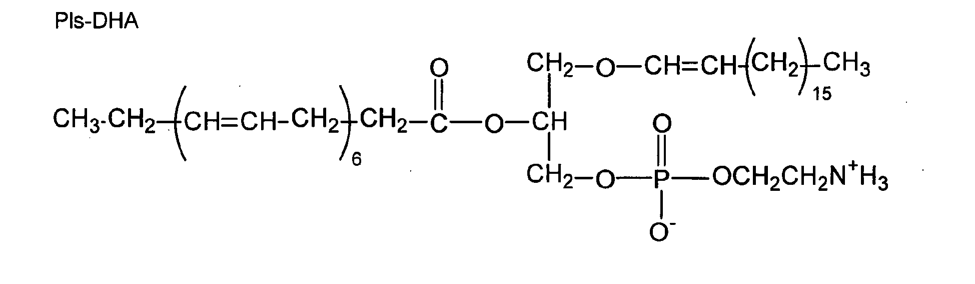

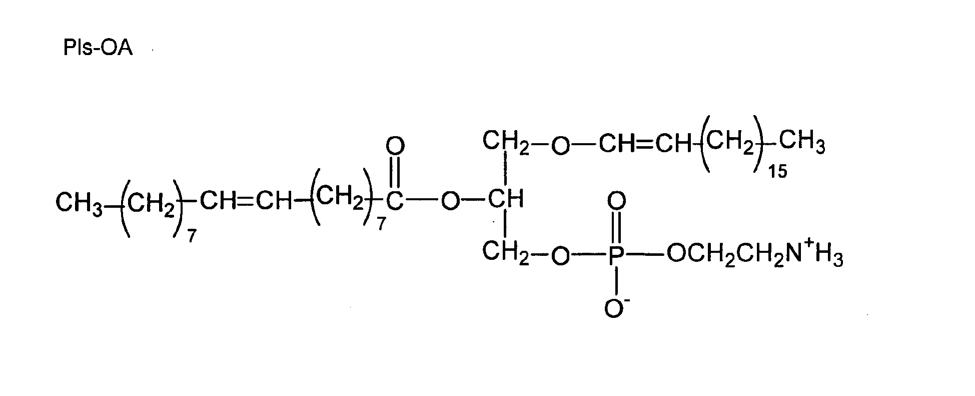

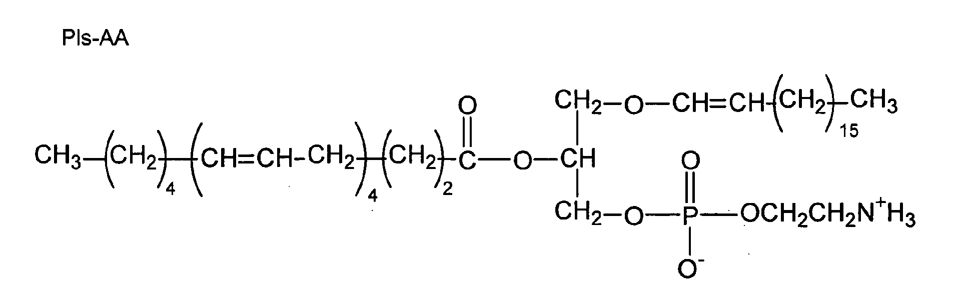

### Example 1: Evaluation of amyloid protein aggregation inhibition induced by a plasmalogen

In this Example, evaluation was carried out by a method involving the use of thioflavine T (Fujiwara, H. et al., Uncaria rhynchophylla, J Neurosci Res 84, 427-33 (2006)). The method is based on the fact that thioflavine T is linked to a β sheet of an amyloid β protein so as to emit fluorescence.

A plasmalogen or a different phospholipid was diluted with a 50 mM potassium phosphate buffer (pH 7.4) and the resulting solution was dispensed into wells of a 96-well plate (25 µl per well) for fluorescence assay. Amyloid β1-42 was dissolved in a 50 mM potassium phosphate buffer (pH 7.4) to a concentration of 20 µM and the resulting solution was dispensed into the wells (25 µl per well). Incubation was carried out at 85 rpm at 37°C for 24 hours, followed by the addition of 6 µM thioflavine T (50 µl per well). After incubation at room temperature for 30 minutes, assay was carried out with a fluorescent microplate reader (Ex: 442 nm; Em: 485 nm). The value obtained with the addition of purified water instead of a plasmalogen or a different phospholipid was designated as 100%. The value obtained for thioflavine T alone was designated as 0%. Then, the aggregation rates for individual cases were calculated. Table 1 shows the results.

**[Table 1]**

| | Aggregation rate (%) | |
|---|---|---|
| | 10 µM | 20 µM |
| Pls-AA | 94 ± 3 | 86 ± 5 |
| Pls-DHA | 47 ± 4 | 46 ± 6 |
| PE-DHA | 91 ± 6 | 91 ± 7 |
| PC-DHA | 97 ± 8 | 86 ± 1 |

Table 1 shows amyloid protein aggregation rates for plasmalogens (Pls-DHA, Pls-AA) each containing DHA or arachidonic acid and two other DHA-containing phospholipids (PE-DHA, PC-DHA) at different concentrations. Values listed in table 1 are each represented by a mean value ± standard deviation (n = 3).

Pls-DHA at a concentration of 10 µM exhibited particularly strong activity of inhibiting amyloid protein aggregation at a level of approximately 50%. Meanwhile, the other DHA-containing phospholipids exhibited weak activity of inhibiting amyloid protein aggregation. The above results indicate that plasmalogens have stronger activity of inhibiting amyloid protein aggregation than the other phospholipids.

### Example 2: Evaluation of amyloid protein degradation induced by plasmalogen (1)

Also in this Example, evaluation was carried out by a method involving the use of thioflavine T as in Example 1.

Amyloid β1-42 was dissolved in a 50 mM potassium phosphate buffer (pH 7.4) to a concentration of 20 µM and the resulting solution was dispensed into wells of a 96-well plate (25 µl per well) for fluorescence assay. Incubation was carried out at 85 rpm at 37°C for 24 hours. A plasmalogen or a different phospholipid was diluted with a 50 mM potassium phosphate buffer (pH 7.4) and the resulting solution was dispensed into the wells (25 µl per well). Incubation was carried out at 1,200 rpm at room temperature for 30 minutes, followed by the addition of 6 µM thioflavine T (50 µl per well). After incubation at room temperature for 30 minutes, assay was carried out with a fluorescent microplate reader (Ex: 442 nm; Em: 485 nm). The value obtained with the addition of purified water instead of a plasmalogen or a different phospholipid was designated as 100%. The value obtained for thioflavine T alone was designated as 0%. Then, the degradation residual rates for individual cases were calculated. Table 2 shows the results.

**[Table 2]**

| | Degradation residual rate (%) | |
|---|---|---|
| | 10 µM | 20 µM |
| Pls-OA | 61 ± 8 | 56 ± 6 |
| Pls-AA | 67 ± 5 | 51 ± 7 |
| Pls-DHA | 37 ± 9 | 29 ± 2 |
| PE-DHA | 85 ± 3 | 97 ± 3 |
| PC-DHA | 92 ± 22 | 82 ± 5 |

Table 2 shows amyloid protein degradation residual rates for three different plasmalogens and two other DHA-containing phospholipids at different concentrations. Values in table 2 are each represented by a mean value ± standard deviation (n = 3).

Pls-DHA at a concentration of 10 µM exhibited strong activity of promoting amyloid protein degradation at a level of approximately 60%. Pls-AA and Pls-OA each at a concentration of 10 µM exhibited activity of promoting amyloid protein degradation at a level of approximately 30% to 40%. The above results indicate that plasmalogens have stronger activity of promoting amyloid protein degradation than the other phospholipids.

### Example 3: Evaluation of amyloid protein degradation induced by plasmalogen (2)

In this Example, evaluation was carried out by a method involving the use of a transmission electron microscope (TEM) (Ono, K. et al., Exp Neurol 189, 380-92 (2004)). TEMs can be used to irradiate a subject with an electron beam, magnify electrons transmitting through the subject, and observe the magnified electrons.

Amyloid β1-42 was dissolved in a 50 mM potassium phosphate buffer (pH 7.4) to a concentration of 20 µM and the resulting solution was dispensed into 0.6-ml Eppendorf tubes (100 µl each). Incubation was carried out at 85 rpm at 37°C for 24 hours. A plasmalogen or a different phospholipid was diluted with a 50 mM potassium phosphate buffer (pH 7.4) and the resulting solution was dispensed into the tubes (100 µl each). Incubation was carried out at 1,200 rpm at room temperature for 30 minutes. The obtained sample (10 µl) was placed on a copper mesh and left to stand for 1 minute, followed by staining with 1% phosphotungstic acid for observation using TEM. Amyloid protein degradation was observed with TEM.

A control to which purified water had been added and the sample to which Pls-DHA had been added were observed at 30,000- and 110,000-fold magnifications.

Amyloid protein degradation in the sample to which Pls-DHA had been added was clearly observed at a low magnification (30,000-fold magnification) and at a high magnification (110,000-fold magnification).

All publications, patents, and patent applications cited herein are incorporated herein by reference in their entirety.

## Claims

1. A composition for promoting amyloid protein degradation, comprising a plasmalogen as an active ingredient.

2. A composition for inhibiting amyloid protein aggregation, comprising a plasmalogen as an active ingredient.

3. The composition according to claim 1 or 2, comprising a plasmalogen having a glycerol skeleton comprising an ester-linked docosahexaenoic acid at the sn-2 position.

4. A composition for preventing or treating diseases associated with amyloidosis, comprising a plasmalogen as an active ingredient.
